# DEMANDE DE BREVET EUROPEEN

(11) **EP 1 051 981 A1**
(43) Date de publication de la demande: **15.11.2000**
(21) Numéro de dépôt: 00401259.7
(22) Date de dépôt: 09.05.2000
(51) Int. Cl.: A61L 2/26, A61B 19/02

(54) **Plateaux de stérilisation superposables et conteneur associé**

(30) Priorité: 12.05.1999 FR 9906071
(71) Demandeur: Medical Conteneur (Groupe Moria-Dugast), 77100 Meaux (FR)
(72) Inventeur: Costes, Xavier, 95470 Fosses (FR)
(74) Mandataire: Jaunez, Xavier

(57) **Abrégé**

L'invention concerne un plateau de stérilisation (1) aménagé pour supporter une instrumentation médicale, constitué par un bac (2) présentant supérieurement deux plats (6) formant poignées qui sont surmontés de plots d'accrochage (20).

Conformément à l'invention, chaque plot d'accrochage (20) est constitué par un pied au moins en partie réalisé en matériau souple et par une tête plus large surmontant ledit pied. Des lumières débouchantes (30) d'un premier type, présentant un resserrement (31) intermédiaire formant un passage à point dur, et des lumières non débouchantes (40) d'un deuxième type présentant une forme de trou de serrure, sont prévus sur le couvercle de recouvrement (10), et de préférence aussi sur le fond (3) du bac (2) pour permettre une solidarisation entre plateaux superposés.

## Description

La présente invention concerne le domaine de l'équipement médical, et plus particulièrement les plateaux de stérilisation aménagés pour supporter une instrumentation médicale.

Pour l'arrière-plan technologique de l'invention, on pourra se référer aux documents US-A-5 340 551, US-A-5 281 400, US-A-5 384 103 et US-A-5 628 970.

De tels plateaux sont couramment utilisés par les chirurgiens ou les personnels d'aide associés pour transporter sur le site opératoire l'instrumentation médicale en rapport avec l'intervention concernée. Le plateau est aménagé pour supporter divers instruments, par exemple des pinces, des endoscopes etc. qui sont plus ou moins fragiles. Ces plateaux doivent être réalisés pour pouvoir supporter, après utilisation, les phases habituelles de nettoyage, conformément à un cycle de décontamination et de stérilisation comme c'est l'usage dans le domaine médical.

L'invention concerne plus particulièrement un plateau de stérilisation aménagé pour supporter une instrumentation médicale, constitué par un bac présentant supérieurement deux plats formant poignées, lesdits plats étant surmontés par des plots en saillie servant à accrocher un couvercle de recouvrement muni à cet effet de lumières associées, conformément au préambule de la revendication 1.

On rencontre ainsi souvent des couvercles plats de forme rectangulaire, à une extrémité desquels on trouve une paire de lumières débouchantes en forme de U, et au voisinage de l'autre extrémité des lumières en forme de trou de serrure. Les deux plats formant poignées sont surmontés par des plots rigides dont la tête élargie permet un accrochage du couvercle au niveau des lumières précitées. Pour le verrouillage, le couvercle de recouvrement est posé sur le dessus du bac, puis glissé longitudinalement de façon que les plots rigides coulissent dans les lumières associées, et en particulier réalisent l'accrochage du couvercle après une course de translation déterminée.

Les plots rigides sont réalisés sous forme de tétons métalliques rivetés ou vissés sur les plats formant poignées, de sorte que même avec un usinage précis des lumières du couvercle, on ne peut parvenir à un maintien véritablement fiable du couvercle dans la position accrochée de celui-ci.

Ces plateaux de stérilisation peuvent être éventuellement superposés, de sorte qu'ils comportent habituellement des pastilles de centrage de façon à être gerbables selon un empilement qui peut être disposé à l'intérieur d'un conteneur. Il a ainsi été proposé des conteneurs présentant un compartiment recevant trois plateaux superposés. Le compartiment est habituellement dimensionné de telle façon que les trois plateaux soient exactement reçus à l'intérieur du compartiment, sans risque de désolidarisation d'un plateau ou du couvercle fermant le plateau supérieur. Néanmoins, à l'ouverture du conteneur, lorsque l'opérateur dégage le plateau supérieur, il se peut que par un mouvement maladroit un choc soit exercé sur le couvercle et que celui-ci glisse sur le bac concerné et ne protège plus l'intérieur du plateau. En outre, il est fréquent que l'opérateur doive manipuler un empilement de plateaux sorti du conteneur, et il faut bien reconnaître que l'absence de solidarisation entre les plateaux superposés induit un risque de glissement relatif et de chute d'un des plateaux, qui risque d'endommager l'instrumentation concernée, avec des conséquences qui peuvent être dramatiques pour le fonctionnement de ces instruments, sans parler du coût inhérent au remplacement de ceux-ci. Le léger centrage procuré par quatre pastilles agencées aux quatre coins du fond des bacs, venant s'insérer dans le contour du plateau inférieur, n'est donc aucunement satisfaisant en terme de verrouillage.

L'invention a pour objet d'améliorer la structure de tels plateaux de stérilisation afin d'éviter les inconvénients précités.

L'invention a ainsi pour but de concevoir un plateau de stérilisation dont la structure permet à la fois un verrouillage fiable de son couvercle de recouvrement, tout en autorisant des combinaisons de superpositions de types variés, avec aussi la possibilité de verrouiller entre eux les plateaux superposés.

Ce problème est résolu conformément à l'invention grâce à un plateau de stérilisation du type précité, dans lequel chaque plat formant poignée est équipé d'au moins un plot d'accrochage constitué par un pied au moins en partie réalisé en matériau souple et par une tête plus large surmontant ledit pied, et dans lequel le couvercle présente en outre à une extrémité au moins une lumière débouchante d'un premier type présentant un resserrement intermédiaire formant un passage à point dur pour le pied du plot d'accrochage concerné, et à son extrémité opposée au moins une lumière non débouchante d'un deuxième type présentant une forme de trou de serrure dont la partie large permet le passage de la tête du plot d'accrochage concerné et la partie étroite le coulissement du pied dudit plot, conformément à la partie caractérisante de la revendication 1.

L'agencement de telles lumières débouchantes à resserrement intermédiaire permet de réaliser, en combinaison avec le caractère déformable du pied des plots d'accrochage, un verrouillage qui s'apparente tout à fait à un encliquetage, procurant une fiabilité optimale.

Conformément à un mode de réalisation particulier, le pied de chaque plot d'accrochage est constitué par une colonnette rigide entourée sur toute sa hauteur d'un manchon en matériau souple, par exemple en silicone, et la tête élargie du plot d'accrochage est d'une pièce avec la colonnette, avec un filetage central pour la fixation dudit plot sur le plat concerné. Ce mode de construction permet de remplacer si nécessaire le manchon en matériau souple en cas d'usure prématurée de celui-ci, tout en conservant la structure de base en général métallique constituant les plots d'accrochage.

Avantageusement, chaque plat du bac est équipé de deux plots d'accrochage, les quatre plots dudit bac étant identiques et positionnés selon un rectangle.

De préférence encore, la ou chaque lumière débouchante du premier type a un resserrement intermédiaire qui est intercalé entre une partie d'entrée dont la largeur diminue en direction dudit resserrement et une partie de fond sensiblement circulaire. En particulier, la partie d'entrée est définie par deux tronçons rectilignes inclinés l'un vers l'autre, se raccordant à deux tronçons rectilignes parallèles qui forment le resserrement intermédiaire.

De préférence alors, la largeur de passage du resserrement intermédiaire est notablement inférieure au diamètre extérieur du pied du plot d'accrochage concerné, et le diamètre de la partie de fond est sensiblement égal audit diamètre extérieur.

Avantageusement encore, la ou chaque lumière non débouchante du deuxième type a une partie large circulaire dont le diamètre est suffisant pour le passage de la tête du plot d'accrochage concerné, et une partie étroite dont la largeur est constante et sensiblement égale au diamètre extérieur du pied dudit plot.

Il est par ailleurs intéressant de prévoir que le couvercle présente à une extrémité une paire de lumières du premier type et à l'autre extrémité une paire de lumières du deuxième type, ces quatre lumières étant positionnées selon un rectangle.

Conformément à un mode de réalisation particulièrement avantageux, le bac a un fond qui est équipé de lumières du premier et du deuxième types, identiques à celles du couvercle, pour permettre une solidarisation avec un autre plateau de stérilisation sous-jacent par les plots d'accrochage de ce dernier. Ceci permet de réaliser des empilements de plateaux de stérilisation superposés parfaitement solidarisés entre eux.

Avantageusement alors, la ou les lumières débouchantes du deuxième type prévues sur le fond du bac se raccordent à une encoche ménagée dans la paroi latérale adjacente dudit bac, ladite encoche ayant une hauteur au moins égale à celle des pions d'accrochage pour permettre une mise en place par glissement d'un plateau sur l'autre. De préférence, les plots des plats et les lumières du fond sont respectivement à l'aplomb l'un de l'autre. Ceci permet d'assurer la verticalité de l'empilement de plateaux de stérilisation superposés.

On peut imaginer que dans des cas extrêmes d'erreur de manipulation d'un opérateur, un choc imprimé au niveau du plateau inférieur de l'empilement transporté à la main risque de désolidariser ce dernier plateau du restant de la pile. Pour éviter un tel risque qui aurait les conséquences fâcheuses déjà mentionnées plus haut, il est alors prévu que le bac soit équipé d'un moyen de verrouillage au niveau de l'une au moins des lumières débouchantes du deuxième type prévues sur le fond dudit bac.

Dans un mode particulier de réalisation, le moyen de verrouillage précité est constitué par une barrette mobile assujettie au bac et coulissant entre une position de dégagement dans laquelle le pion d'accrochage du plateau sous-jacent peut pénétrer dans ou sortir de la lumière débouchante, et une position de verrouillage dans laquelle ledit pion d'accrochage est bloqué dans ladite lumière débouchante.

La structure exposée plus haut du plateau de stérilisation et du couvercle est parfaitement compatible avec une réalisation modulaire avec des demi-composants.

On pourra ainsi prévoir que le plateau de stérilisation est agencé sous forme d'un demi-plateau surplombant un plateau et accroché à celui-ci, le recouvrement se faisant par au moins un demi-couvercle ou un couvercle accroché par les plots d'accrochage concernés.

L'invention concerne également un conteneur de stérilisation, du type comportant un compartiment destiné à recevoir une pluralité de plateaux de stérilisation superposés, dont le plateau supérieur est recouvert par un couvercle, ledit conteneur étant remarquable en ce que les plateaux présentent l'une au moins des caractéristiques précitées, et que le compartiment a un dimensionnement qui correspond à l'encombrement desdits plateaux.

D'autres caractéristiques et avantages de l'invention apparaîtront à la lumière de la description qui va suivre et des dessins annexés, concernant un mode de réalisation particulier, en référence aux figures où :
- la figure 1 est une vue en perspective éclatée illustrant un plateau de stérilisation conforme à l'invention, avec un bac surmonté de son couvercle de recouvrement;
- la figure 2 est une vue en coupe selon le plan II de la figure 1, montrant un détail à plus grande échelle au niveau de l'axe d'un des plots d'accrochage équipant les plats du bac ;
- la figure 3 est une vue de dessous du couvercle de recouvrement précité, et les figures 4 et 5 illustrent en coupe, à plus grande échelle, les détails IV et V correspondant respectivement à une lumière débouchante du premier type ( à resserrement intermédiaire) et une lumière non débouchante du deuxième type (en forme de trou de serrure) ;
- la figure 6 est une vue en perspective éclatée illustrant la superposition de deux plateaux de stérilisation, avec un verrouillage mutuel entre les deux bacs superposés grâce aux lumières du premier et du deuxième types également prévues sur le fond du bac supérieur ;
- la figure 7 est une vue de dessus de ce dernier bac, dont le fond est équipé des lumières spéciales de verrouillage ;
- les figures 8 et 9 sont des coupes de détail à plus grande échelle selon VIII-VIII et IX-IX de la figure 7, le plan de coupe passant par l'axe des plots d'accrochage ;
- les figures 10a et 10b sont des vues partielles d'une variante du plateau de stérilisation précédent, à fond équipé de lumières, avec un moyen supplémentaire de verrouillage au niveau de l'une au moins des lumières débouchantes, respectivement en position de déverrouillage et de verrouillage ;
- la figure 11 est une vue en coupe illustrant un conteneur de stérilisation conforme à l'invention, ici équipé de trois plateaux superposés, dont le plateau supérieur est fermé par un couvercle de recouvrement, conformément à la réalisation précédemment illustrée.

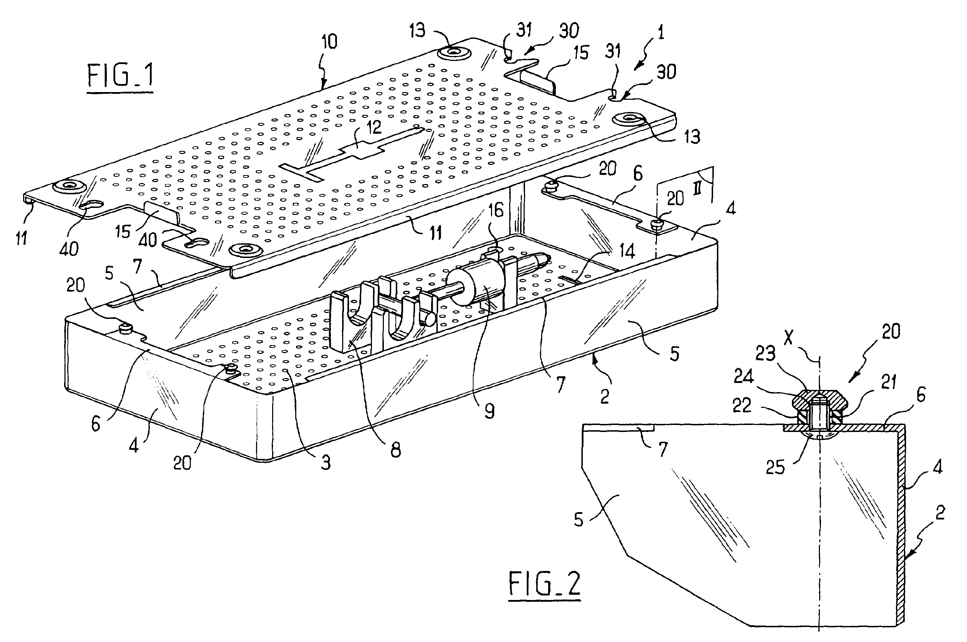

Sur la figure 1, on distingue un plateau de stérilisation 1 conforme à l'invention, qui est aménagé pour supporter une instrumentation médicale. Ce plateau est constitué par un bac 2, ici de forme rectangulaire, constitué par un fond 3 auquel se raccordent des parois latérales verticales 4 et 5. Le fond 3 présente classiquement des perforations pour permettre un nettoyage correct dans le cadre d'un cycle de décontamination et de stérilisation classique. Ce fond 3 est équipé de différent inserts 8, en général en matière plastique tel que du téflon, qui permettent de supporter une instrumentation médicale 9. L'illustration donnée ici ne constitue naturellement qu'un exemple, dans la mesure où les instruments pourront être de type très divers, et où le bac contiendra une pluralité d'instruments, en général associés à un type particulier d'intervention chirurgicale. Au niveau des parois latérales 4 correspondant à la largeur du bac, on trouve deux plats 6 tournés vers l'intérieur qui forment deux poignées. Ces plats 6 sont surmontés par des plots 20 en saillie servant à accrocher un couvercle de recouvrement 10 qui est muni à cet effet de lumières associées. Le couvercle 10, présentant également des perforations pour la même raison que celle précédemment donnée, comporte en général un dessin schématique 12 qui permet de repérer immédiatement de l'extérieur, lorsque le plateau de stérilisation est fermé par son couvercle, les instruments qui sont contenus dans le bac. Le dessin 12 facilite également la remise en place des instruments, après utilisation, dans le plateau aménagé. Le couvercle 10 présente en outre inférieurement deux ailes longitudinales 11, qui facilitent la mise en place du couvercle 10 sur le bac 2, à la façon d'un tiroir coulissant.

On retrouve également des moyens bien connus associés au gerbage de plateaux éventuellement munis d'un couvercle. On trouve ainsi deux fentes 14 pratiquées dans le fond 3 du bac 2, et deux rabats verticaux 15 homologues prévus au niveau d'échancrures latérales du couvercle 10. Le fond 3 du bac est équipé en outre de quatre pastilles 16 convexes, c'est-à-dire bombées vers le bas, et le couvercle 10 présente quatre cuvettes 13 permettant également un gerbage en posant un élément sur ledit couvercle.

Conformément à une première caractéristique de l'invention, chaque plat formant poignée 6 est équipé d'au moins un plot d'accrochage 20 constitué par un pied au moins en partie réalisé en matériau souple et par une tête plus large surmontant ledit pied. La structure des plots d'accrochage 20 sera mieux comprise en se référant à la figure 2.

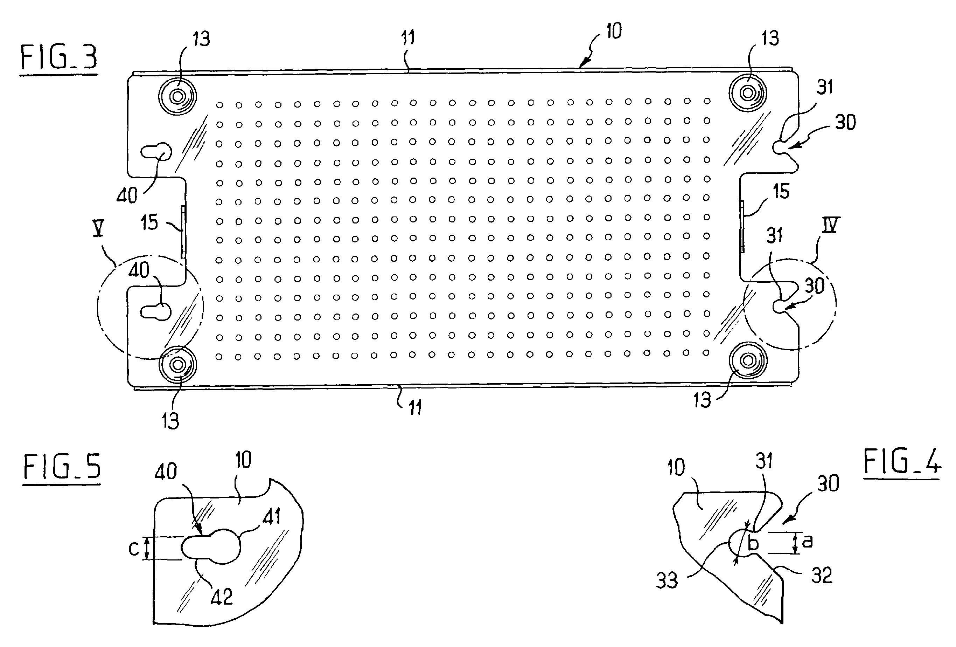

En l'espèce, le pied du plot d'accrochage 20 est constitué par une colonnette rigide 21 entourée sur toute sa hauteur d'un manchon 22 en matériau souple , par exemple en silicone. La tête élargie 23 du plot d'accrochage 20 est ici d'une pièce avec la colonnette 21, en formant ainsi une pièce épaulée, avec un filetage central 24 pour la fixation dudit plot sur le plat concerné 6 au moyen d'une vis 25. Ce manchon ou bague 22 en matériau souple est susceptible d'une déformation radiale lors de l'accrochage du couvercle 10 au niveau des lumières débouchantes associées. On pourrait naturellement prévoir en variante un pied de plot d'accrochage entièrement réalisé en matériau souple, cependant le mode de réalisation illustré ici permet d'avoir à la fois une fixation rigide par les parties métalliques du plot, et un remplacement possible et aisé du manchon en matériau souple 22 qui constitue une pièce d'usure. Les lumières débouchantes associées prévues sur le couvercle de recouvrement 10 ont une géométrie particulière qui sera mieux comprise en se référant aux figures 3 à 5.

Le couvercle 10 présente en effet à une extrémité au moins une lumière débouchante 30 d'un premier type présentant un resserrement 31 intermédiaire formant un passage à point dur pour le pied du plot d'accrochage 20 concerné, et à son extrémité opposée au moins une lumière non débouchante 40 d'un deuxième type présentant une forme de trou de serrure dont la partie large permet le passage de la tête du plot d'accrochage concerné et la partie étroite le coulissement dudit plot. La présence du resserrement intermédiaire 31 formant passage à point dur est extrêmement avantageuse dans la mesure où elle permet de réaliser un verrouillage efficace avec la sensation précise pour l'opérateur que la position de verrouillage, avec butée au fond des lumières concernées, est bien atteinte.

En l'espèce, la lumière débouchante du premier type 30 a un resserrement intermédiaire 31 qui est intercalé entre une partie d'entrée 32 dont la largeur diminue en direction dudit resserrement et une partie de fond 33 sensiblement circulaire. Cette partie d'entrée 32 constitue ainsi un couloir de guidage pour le pied du plot d'accrochage 20. Il convient toutefois de noter que la présence des ailes longitudinales 11 sur le couvercle de recouvrement 10 participe déjà largement au guidage dans la direction longitudinale lors de la mise en place et du verrouillage du couvercle. Dans la position finale d'accrochage, le pied du plot d'accrochage est reçu dans la partie de fond 33 sensiblement circulaire, dont le diamètre sera donc proche de celui dudit pied. En l'espèce, la partie d'entrée 32 est définie par deux tronçons rectilignes inclinés l'un vers l'autre, se raccordant à deux tronçons rectilignes parallèles qui forment le resserrement intermédiaire 31. La réalisation d'un tel resserrement intermédiaire 31 en deux tronçons rectilignes parallèle permet d'avoir un serrage efficace du manchon 22 en matériau souple du plot d'accrochage 20 concerné tout en évitant un effet de coupure dudit manchon que pourrait par exemple provoquer un resserrement réalisé par un angle vif. La largeur de passage a du resserrement intermédiaire 31 est notablement inférieure au diamètre extérieur du pied du plot d'accrochage 20 concerné, de façon à réaliser l'effet de point dur recherché, et le diamètre b de la partie de fond 33 est sensiblement égal audit diamètre extérieur.

La lumière non débouchante du deuxième type 40 a une forme qui était déjà utilisée dans les plateaux de stérilisation connus, mais le dimensionnement de celle-ci est toutefois choisi en rapport avec la structure particulière des plots d'accrochage dont le pied est au moins en partie réalisé en matériau souple. La lumière non débouchante du deuxième type 40 présente ainsi une partie large 41 de forme circulaire, dont le diamètre est suffisant pour permettre le passage de la tête 23 du plot d'accrochage concerné 20, et une partie étroite 42 formant lumière oblongue à bords parallèles et à fond demi-circulaire, dont la largeur c est constante et sensiblement égale au diamètre extérieur du pied dudit plot.

En position de mise en place, la tête 23 des deux plots 20 associés aux lumières 40 sont passées dans la partie large 41 associée, les deux autres plots étant alors disposés au niveau de la partie d'entrée 32 des lumières débouchantes du premier type 30. Après glissement du couvercle 10, qui est en appui sur les ailes 7 et les plats 6 du bac 2, les pieds des pions d'accrochage arrivent ainsi respectivement au fond de la partie étroite 42 pour les lumières non débouchantes du deuxième type 40, et dans la partie de fond 33 pour les lumières débouchantes du premier type 30.

A titre d'exemple, pour un diamètre extérieur de 10 millimètres du manchon souple équipant chaque plot d'accrochage 20, on pourra choisir une largeur c de 9,5 millimètres pour la partie étroite 42 de la lumière non débouchante 40, puis, pour la lumière débouchante 30, un resserrement intermédiaire dont la largeur a est de 9 millimètres, et une partie de fond circulaire 33 d'un diamètre b de 10 millimètres.

En l'espèce, chaque plat 6 du bac 2 est équipé de deux plots d'accrochage 20, les quatre plots dudit bac étant identiques et positionnés selon un rectangle. De même, le couvercle 10 présente à une extrémité une paire de lumières du premier type 30, et à l'autre extrémité une paire de lumières du deuxième type 40, ces quatre lumières étant positionnées selon un rectangle.

Il est toutefois possible de prévoir une conception modulaire plus élaborée, avec des demi-bacs et/ou des demi-couvercles. Dans ce cas, un demi-bac ou un demi-couvercle ne présentera respectivement qu'un pion d'accrochage à chaque extrémité ou qu'une lumière de chaque type à chaque extrémité.

Il est évidemment intéressant de prévoir la possibilité d'un verrouillage entre des plateaux superposés, lorsque l'on utilise plusieurs plateaux, non seulement pour leur stockage dans un conteneur, mais aussi pour leur manipulation comme un tout unitaire permettant de véhiculer plusieurs instrumentations médicales complémentaires.

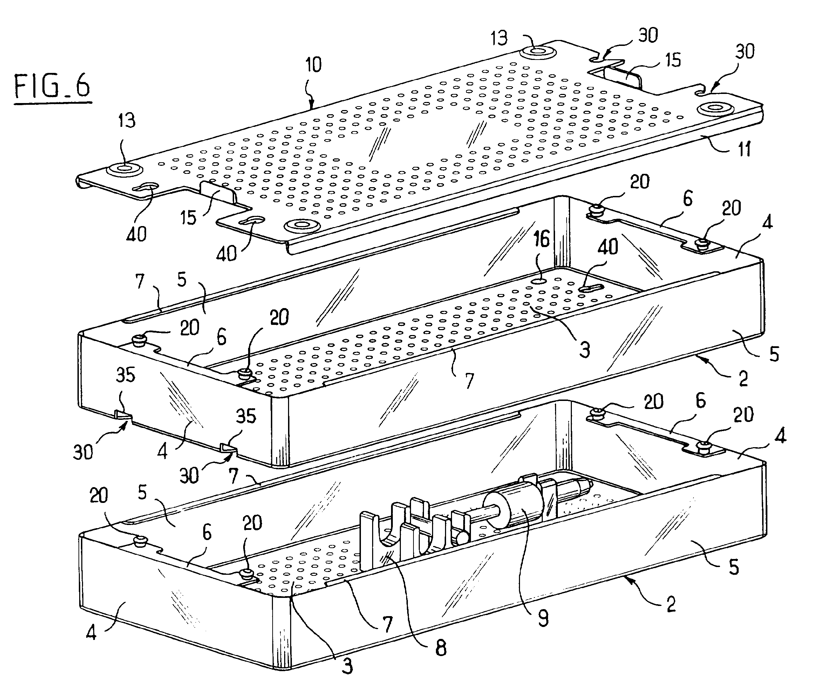

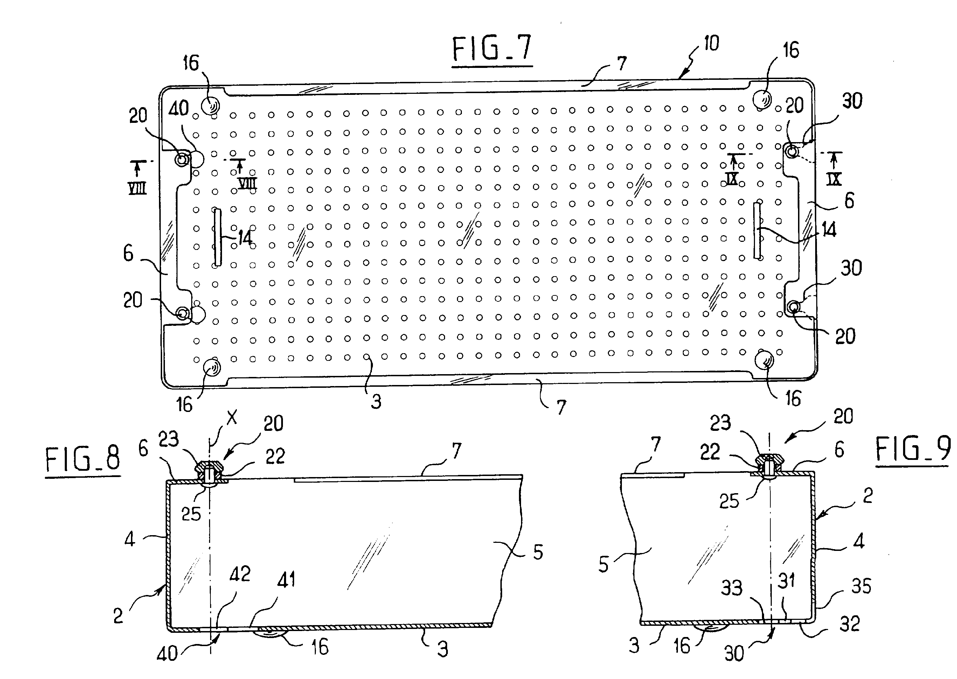

La figure 6 illustre ainsi une telle possibilité, et l'on constate que le bac supérieur, qui est inséré entre le bac inférieur et le couvercle de recouvrement 10, a un fond 3 qui est équipé de lumières du premier et du deuxième types qui sont identiques à celles du couvercle 10 déjà décrit, pour permettre une solidarisation avec l'autre plateau de stérilisation sous-jacent par les plots d'accrochage 20 de ce dernier. Un tel agencement du plateau de stérilisation sera mieux compris en se référant aux figures 7 à 9.

On retrouve ainsi, d'un côté du fond 3, une paire de lumières débouchantes 30 du premier type, c'est-à-dire à resserrement intermédiaire formant un passage à point dur pour le pied d'accrochage du plot d'accrochage concerné, et de l'autre côté une paire de lumières non débouchantes 40 du deuxième type présentant une forme de trou de serrure. Les plots 20 des plats 6 et les lumières 30, 40 du fond 3 sont respectivement à l'aplomb l'un de l'autre, comme cela est mieux visible sur les coupes des figures 8 et 9. Ainsi, lorsque plusieurs plateaux sont superposés, on crée, dans la position de verrouillage, un empilement qui est parfaitement vertical.

Par ailleurs, ainsi que cela est mieux visible sur les figures 6 et 9, il est avantageusement prévu que les lumières débouchantes du deuxième type 30 prévues sur le fond 3 du bac se raccordent à une encoche 35 ménagée dans la paroi latérale adjacente 4 dudit bac, ladite encoche ayant une hauteur au moins égale à celle des pions d'accrochage 20 pour permettre une mise en place d'un plateau sur l'autre par coulissement.

Toutefois, il peut arriver que dans certaines situations de manipulation extrême, le plateau inférieur d'un empilement de plateaux mutuellement accrochés vienne à subir un choc latéral, qui pourrait provoquer sa désolidarisation du reste de l'empilement. Bien que ceci ait peu de chances de se produire dans la mesure où le serrage à point dur procure déjà une très grande sécurité, on pourra éventuellement prévoir une mesure supplémentaire de sécurité.

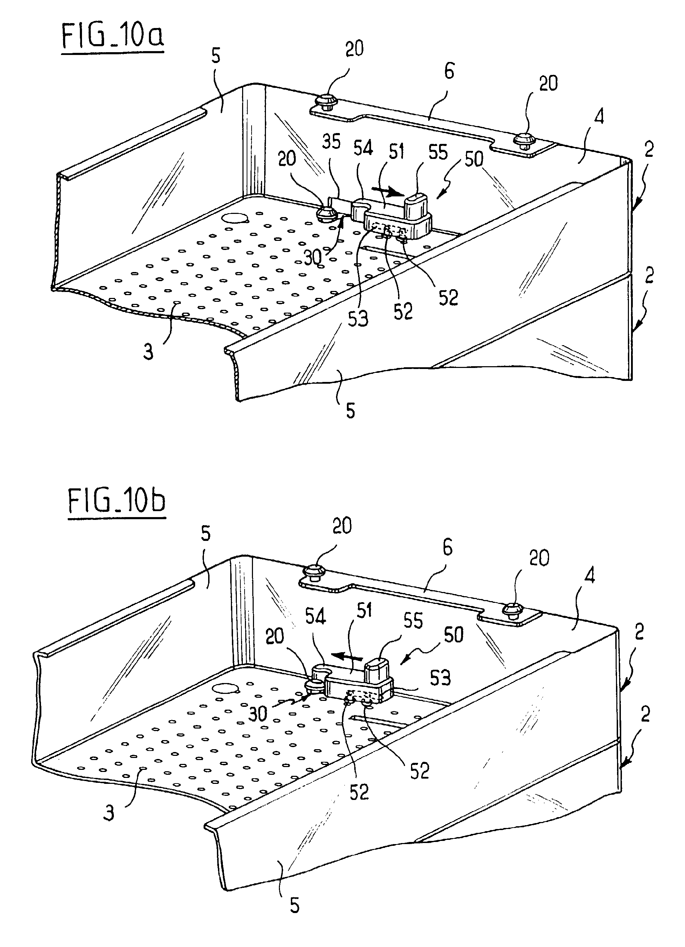

Ceci est illustré aux figures 10a et 10b, où l'on a prévu que le bac 2 est équipé d'un moyen de verrouillage 50 au niveau de l'une au moins des lumières débouchantes du premier type 30 prévues sur le fond 3 dudit bac. En l'espèce, le moyen de verrouillage est constitué par une barrette mobile 50 qui est assujettie au fond du bac 3 par deux vis 52 dont les tiges passent dans une lumière oblongue 53 dudit fond 3. La barrette 50 comporte une partie principale d'assujettissement 51 surmontée d'une partie de manoeuvre 55, et un becquet 54 qui vient, en position de verrouillage, s'insérer entre le pion d'accrochage 20 du plateau inférieur, et la paroi latérale 4 du plateau supérieur.

La barrette mobile 50 peut ainsi coulisser entre une position de dégagement (figure 10a) dans laquelle le pion d'accrochage 20 du plateau sous-jacent peut pénétrer ou sortir de la lumière débouchante 30, et une position de verrouillage (figure 10b) dans laquelle ledit pion d'accrochage est bloqué dans ladite lumière débouchante.

On pourra bien entendu prévoir d'autres variantes de tels moyens de verrouillage, étant entendu que la structure de tels moyens devra être compatible avec les exigences de nettoyage et de propreté, sans non plus constituer une gêne pour la mise en place de l'instrumentation médicale sur le fond du bac, ou la manipulation par l'opérateur insérant ses doigts sous les plats 6 formant poignées pour retirer le plateau d'un conteneur ou transporter ledit plateau d'un endroit à un autre.

Cette solidarisation entre plateaux de stérilisation superposés, qui n'a jamais été rencontrée dans les réalisations de l'art antérieur, est extrêmement intéressante dans la pratique, notamment pour le logement en bloc de tels plateaux à l'intérieur d'un conteneur de stérilisation associé.

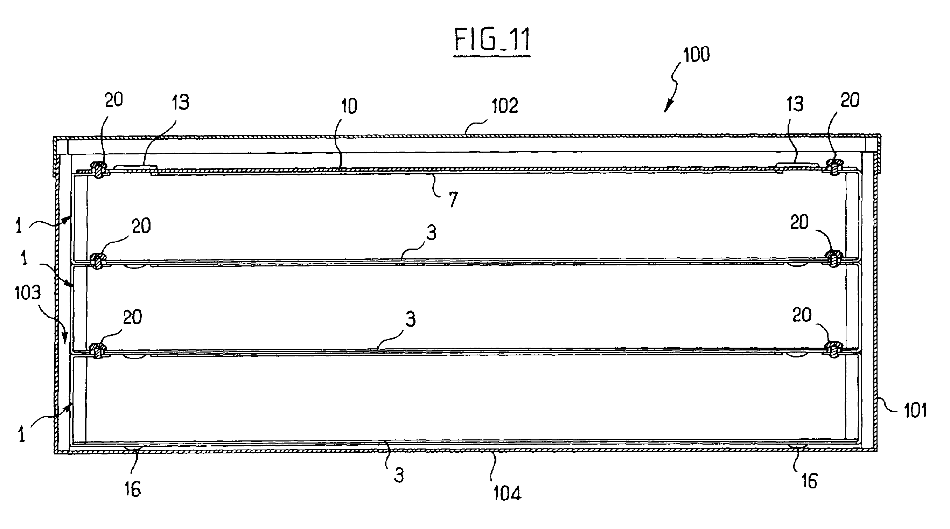

Une telle implantation dans un conteneur de stérilisation est illustré sur la figure 11.

On distingue un conteneur ayant la forme d'un boîtier 100, dont la partie 101 a un fond 104 et est surmontée d'un couvercle de fermeture 102, éventuellement articulé, avec un compartiment principal 103 destiné à recevoir une pluralité de plateaux de stérilisation superposés, dont le plateau supérieur est recouvert par un couvercle. En l'espèce, le compartiment 103 correspond à l'ensemble de l'espace disponible à l'intérieur du conteneur 100, mais il va de soi que l'on pourra prévoir des logements mixtes, avec un espace 103 qui ne constitue qu'une partie seulement de cet espace, le restant étant dédié à l'implantation d'autres instruments, ainsi que cela est connu de le prévoir. Les plateaux 1, ici au nombre de trois, sont réalisés conformément à la description qui précède, en étant verrouillés l'un à l'autre, pour former un ensemble unitaire de trois plateaux superposés recouvert supérieurement d'un couvercle 10. Le compartiment 103 a un dimensionnement qui correspond à l'encombrement des plateaux de stérilisation 1, ce qui permet à la fois de respecter les règles habituelles en matière de stérilisation (par exemple avec la mise en place d'une enveloppe textile autour de l'empilement de plateaux), et d'éviter tout risque de désolidarisation de l'un des plateaux lors d'un transport un peu brutal.

L'invention n'est pas limitée aux modes de réalisation qui viennent d'être décrits mais englobe au contraire toute variante reprenant, avec des moyens équivalents, les caractéristiques essentielles énoncées plus haut.

## Revendications

1. Plateau de stérilisation aménagé pour supporter une instrumentation médicale, constitué par un bac (2) présentant supérieurement deux plats (6) formant poignées, lesdits plats étant surmontés par des plots en saillie servant à accrocher un couvercle de recouvrement (10) muni à cet effet de lumières associées, caractérisé en ce que :
- chaque plat formant poignée (6) est équipé d'au moins un plot d'accrochage (20) constitué par un pied (21,22) au moins en partie réalisé en matériau souple et par une tête plus large (23) surmontant ledit pied ;
- le couvercle (10) présente à une extrémité au moins une lumière débouchante (30) d'un premier type présentant un resserrement (31) intermédiaire formant un passage à point dur pour le pied du plot d'accrochage concerné, et à son extrémité opposée au moins une lumière non débouchante (40) d'un deuxième type présentant une forme de trou de serrure dont la partie large permet le passage de la tête du plot d'accrochage concerné et la partie étroite le coulissement du pied dudit plot.

2. Plateau de stérilisation selon la revendication 1, caractérisé en ce que le pied (21,22) de chaque plot d'accrochage (20) est constitué par une colonnette rigide (21) entourée sur toute sa hauteur d'un manchon (22) en matériau souple, par exemple en silicone.

3. Plateau de stérilisation selon la revendication 2, caractérisé en ce que la tête élargie (23) du plot d'accrochage (20) est d'une pièce avec la colonnette (21), avec un filetage central (24) pour la fixation dudit plot sur le plat concerné (6).

4. Plateau de stérilisation selon l'une des revendications 1 à 3, caractérisé en ce que chaque plat (6) du bac (2) est équipé de deux plots d'accrochage (20), les quatre plots (20) dudit bac étant identiques et positionnés selon un rectangle.

5. Plateau de stérilisation selon l'une des revendications 1 à 4, caractérisé en ce que la ou chaque lumière débouchante du premier type (30) a un resserrement intermédiaire (31) qui est intercalé entre une partie d'entrée (32) dont la largeur diminue en direction dudit resserrement et une partie de fond (33) sensiblement circulaire.

6. Plateau de stérilisation selon la revendication 5, caractérisé en ce que la partie d'entrée (32) est définie par deux tronçons rectilignes inclinés l'un vers l'autre, se raccordant à deux tronçons rectilignes parallèles qui forment le resserrement intermédiaire (31).

7. Plateau de stérilisation selon la revendication 5 et la revendication 6, caractérisé en ce que la largeur de passage du resserrement intermédiaire (31) est notablement inférieure au diamètre extérieur du pied (21,22) du plot d'accrochage (20) concerné, et le diamètre de la partie de fond (33) est sensiblement égal audit diamètre extérieur.

8. Plateau de stérilisation selon l'une des revendications 1 à 7, caractérisé en ce que la ou chaque lumière non débouchante du deuxième type (40) a une partie large (41) circulaire dont le diamètre est suffisant pour le passage de la tête (23) du plot d'accrochage (20) concerné, et une partie étroite (42) dont la largeur est constante et sensiblement égale au diamètre extérieur du pied (21,22) dudit plot.

9. Plateau de stérilisation selon l'une des revendications 1 à 8, caractérisé en ce que le couvercle (10) présente à une extrémité une paire de lumières du premier type (30) et à l'autre extrémité une paire de lumières du deuxième type (40), ces quatre lumières étant positionnées selon un rectangle.

10. Plateau de stérilisation selon l'une des revendications 1 à 9, caractérisé en ce que le bac (2) a un fond (3) qui est équipé de lumières du premier et du deuxième types (30,40), identiques à celles du couvercle (10), pour permettre une solidarisation avec un autre plateau de stérilisation sous-jacent par les plots d'accrochage (20) de ce dernier.

11. Plateau de stérilisation selon la revendication 10, caractérisé en ce que la ou les lumières débouchantes du deuxième type (30) prévues sur le fond (3) du bac se raccordent à une encoche (35) ménagée dans la paroi latérale adjacente (4) dudit bac, ladite encoche ayant une hauteur au moins égale à celle des pions d'accrochage (20) pour permettre une mise en place par glissement d'un plateau sur l'autre.

12. Plateau de stérilisation selon la revendication 10 ou la revendication 11, caractérisé en ce que les plots (20) des plats (6) et les lumières (30,40) du fond (3) sont respectivement à l'aplomb l'un de l'autre.

13. Plateau de stérilisation selon l'une des revendications 10 à 12, caractérisé en ce que le bac (2) est un équipé d'un moyen de verrouillage (50) au niveau de l'une au moins des lumières débouchantes du deuxième type (30) prévues sur le fond (3) dudit bac.

14. Plateau de stérilisation selon la revendication 13, caractérisé en ce que le moyen de verrouillage est constitué par une barrette mobile (50) assujettie au bac (2) et coulissant entre une position de dégagement dans laquelle le pion d'accrochage (20) du plateau sous-jacent peut pénétrer dans ou sortir de la lumière débouchante (30), et une position de verrouillage dans laquelle ledit pion d'accrochage est bloqué dans ladite lumière débouchante.

15. Plateau de stérilisation selon l'une des revendications 10 à 14, agencé sous forme d'un demi-plateau surplombant un plateau et accroché à celui-ci, le recouvrement se faisant par au moins un demi-couvercle ou un couvercle accroché par les plots d'accrochage (20) concernés.

16. Conteneur de stérilisation (100) du type comportant un compartiment (103) destiné à recevoir une pluralité de plateaux de stérilisation superposés (1), dont le plateau supérieur est recouvert par un couvercle (10), caractérisé en ce que les plateaux (1) sont selon l'une des revendications 1 à 9 et l'une des revendications 10 à 15, et que le compartiment (103) a un dimensionnement qui correspond à l'encombrement desdits plateaux.
